# EUROPEAN PATENT APPLICATION

(11) **EP 4 321 166 A1**
(43) Date of publication of application: **14.02.2024**
(21) Application number: 22784891.8
(22) Date of filing: 04.04.2022
(51) Int. Cl.: A61K 35/747, A61K 38/01, A61P 31/04, A23L 33/135, A23L 33/18, A23K 10/16, A23K 20/147

(54) **COMPOSITION FOR INHIBITING BIOFILM COMPRISING LACTOBACILLUS RHAMNOSUS AS ACTIVE INGREDIENT**

(30) Priority: 05.04.2021 KR 20210043973
(71) Applicant: Vixxol Corporation, Gunpo-si, Gyeonggi-do 15807 (KR); Chr. Hansen A/S, 2970 Hoersholm (DK)
(72) Inventor: YOO, Sung Chul, Siheung-si Gyeonggi-do 14967 (KR); KIM, Seon Hwa, Siheung-si Gyeonggi-do 14977 (KR); KIM, Dong Yeop, Jeonju-si Jeollabuk-do 54970 (KR)
(74) Representative: COPA Copenhagen Patents
(86) International application number: PCT/KR2022/004809
(87) International publication number: WO 2022/215981

(57) **Abstract**

The present invention relates to a composition for inhibiting biofilm comprising at least one selected from the group consisting of *Lactobacillus rhamnosus,* its culture medium, its extract and its cell-free supernatant and collagen hydrolysate. Although *Lactobacillus rhamnosus* can inhibit biofilm formation even as a single agent, the inhibitory ability is significantly increased when it is simultaneously treated with the collagen hydrolysate. The collagen hydrolysate inhibits acid production of *Lactobacillus rhamnosus* or *Streptococcus mutans.* Thus, the *Lactobacillus rhamnosus* and collagen hydrolysate can be usefully used as a composition for inhibiting the production of biofilms, in particular biofilms occurring in the oral cavity and a composition for preventing, alleviating, or treating oral diseases caused by the biofilms.

## Description

### [Technical Field]

The present invention relates to a composition for inhibiting biofilm including *Lactobacillus rhamnosus* and collagen hydrolysate.

### [Background Art]

A biofilm is a film that is generated at the part infected with or attached to a microorganism, which is a film that is wrapped with a polymer matrix and forms a microbial complex produced by microorganisms and is also called biological slime or pellicle. Microorganisms form a biofilm, which is a microbial community, in order to adapt to various environments and live. These biofilms are important for the survival of microorganisms because they not only act as a protective film for microorganisms but also share metabolism by meeting different microorganisms and acquire beneficial properties through gene transfer. That is, the biofilm is an aggregate of various bacteria and at the same time serves as a protective film for the bacterial population, so it is a direct or indirect cause of various diseases caused by the bacteria.

In particular, oral disease is one of the diseases caused by biofilm production, and it can be largely divided into dental caries and other oral diseases. Dental caries is one of the representative infectious diseases caused by infectious bacteria, and it is a disease in which teeth are gradually demineralized by the action of bacteria growing on a plaque, a biofilm produced on teeth. In general, bacteria break down sugar to secrete acid, which causes demineralization of the enamel of the tooth surface, thereby causing dental caries. In addition, gingivitis and periodontitis are also diseases caused by bacterial infection, and the oral bacterial membrane plays an important role.

It is known that about 1,000 types of bacteria exist in the oral cavity, but the most important early caries-causing bacteria is generally known as *Streptococcus* spp. Among them, *Streptococcus mutans* (*S. mutans*) attaches to the tooth surface, and the formation of a biofilm (a bacterial film) that causes dental caries begins. It is known that, in general, *Streptococcus mutans* is the most important to play a role in early biofilm production.

Conventionally, antibacterial agents and the like have been mainly used as a method for preventing or alleviating oral diseases such as dental caries, gingivitis, periodontitis, and bad breath. However, these weaken oral immunity by removing not only oral pathogens but also beneficial bacteria in the oral cavity. Further, antibacterial agents increase resistance due to genetic mutation of oral pathogens, so there is a limit to using them as a method of preventing or alleviating oral diseases caused by oral pathogens.

Therefore, there is a need for the development of a technology that can effectively inhibit the biofilm produced by the *Streptococcus mutans.*

### [Disclosure]

### [Technical Problem]

The present inventors studied a method for effectively inhibiting the biofilm produced by *Streptococcus mutans* and confirmed that a specific strain and collagen hydrolyzate are simultaneously treated to effectively inhibit biofilm production and acid production by the strain, thereby completing the present invention.

Therefore, an object of the present invention is to provide a composition for inhibiting biofilm.

Another object of the present invention is to provide an oral composition for inhibiting biofilm production.

Yet another object of the present invention is to provide a pharmaceutical composition for preventing or treating oral diseases caused by *Streptococcus mutans.*

Still another object of the present invention is to provide a food composition for preventing or alleviating oral diseases caused by *Streptococcus mutans.*

Even another object of the present invention is to provide a feed additive composition for inhibiting biofilm production.

Another object of the present invention is to provide a method for inhibiting biofilm.

Another object of the present invention is to provide a method for treating globular disease caused by *Streptococcus mutans.*

### [Technical Solution]

In order to achieve an object, the present invention provides a composition for inhibiting biofilm, the composition including at least one selected from the group consisting of *Lactobacillus rhamnosus,* its culture medium, its extract and its cell-free supernatant; and collagen hydrolysate.

In order to achieve another object, the present invention provides an oral composition for inhibiting biofilm production, the composition including at least one selected from the group consisting of *Lactobacillus rhamnosus,* its culture medium, its extract and its cell-free supernatant; and collagen hydrolysate.

In order to achieve yet another object, the present invention provides a pharmaceutical composition for preventing or treating oral diseases caused by *Streptococcus mutans,* the composition including at least one selected from the group consisting of *Lactobacillus rhamnosus,* its culture medium, its extract and its cell-free supernatant; and collagen hydrolysate.

In order to achieve still another object, the present invention provides a food composition for preventing or alleviating oral diseases caused by *Streptococcus mutans,* the composition including at least one selected from the group consisting of *Lactobacillus rhamnosus,* its culture medium, its extract and its cell-free supernatant; and collagen hydrolysate.

In order to achieve even another object, the present invention provides a feed additive composition for inhibiting biofilm production, the composition including at least one selected from the group consisting of *Lactobacillus rhamnosus,* its culture medium, its extract and its cell-free supernatant; and collagen hydrolysate.

In addition, in order to achieve the above other object, the present invention provides a method for inhibiting biofilm comprising the step of administering to an individual at least one selected from the group consisting of *Lactobacillus rhamnosus,* its culture medium, its extract and its cell-free supernatant; and collagen hydrolysate.

In addition, in order to achieve the above further object, the present invention provides a method for treating globular disease caused by *Streptococcus mutans* comprises the step of administering to an individual at least one selected from the group consisting of *Lactobacillus rhamnosus,* its culture medium, its extract and its cell-free supernatant; and collagen hydrolysate.

### [Advantageous Effects]

The present invention confirms the synergistic effect of simultaneous treatment of *Lactobacillus rhamnosus* and collagen hydrolysate in inhibiting biofilm production generated by *Streptococcus mutans.* According to the present invention, although *Lactobacillus rhamnosus* can inhibit the biofilm formation even as a single agent, the inhibitory ability is significantly increased when it is simultaneously treated with the collagen hydrolysate. The collagen hydrolysate inhibits acid production of *Lactobacillus rhamnosus* or *Streptococcus mutans.* Thus, the *Lactobacillus rhamnosus* and collagen hydrolysate can be usefully used as a composition for inhibiting the production of biofilms, in particular biofilms occurring in the oral cavity and a composition for preventing, alleviating or treating oral diseases caused by the biofilms.

### [Description of Drawings]

FIG. 1 is a view showing the growth curves of the isolated *Lactobacillus rhamnosus* GG DSM 33156 (LGG) and *Streptococcus mutans* (*S. mutans*)*.*
FIG. 2 is an image of a hydroxyapatite disc (sHA) coated with saliva containing LGG.
FIG. 3 shows that LGG and S. *mutans* in the sHA cultured for 42 hours as a single or dual in which FIG. 3A is a view showing the number of viable cells (colony-forming unit, CFU) of each experimental group, and FIG. 3B is a view showing the dry weight of the biofilm generated in each experimental group.
FIG. 4 is a view confirming the effect of LGG inoculum amount and collagen hydrolysate on the number of viable cells, dry weight, and pH of each strain.
FIG. 5 is a view confirming the effect of collagen hydrolysate on the acid production of S. *mutans* and LGG.
FIG. 6 is a view confirming the concentration dependence in the effect of the collagen hydrolysate on the pH.
FIG. 7 is a view confirming the effect of collagen hydrolysate on the growth of S. *mutans* (SM), which is gram-positive bacteria and caries bacteria or *Streptococcus oralis* (*S. oralis* (SO)), which is its symbiotic bacteria, and LGG.
FIG. 8 is a view confirming a competitive relationship of LGG and S. *mutans* and S. *oralis* which are treated with collagen hydrolyzate by concentration.
FIG. 9 is a view showing an experimental procedure for generating a multi-species biofilm model.
FIG. 10 is a view confirming the change in the S. *mutans* and S. *oralis* population according to the LGG inoculum amount.
FIG. 11 is a view confirming the effect of the collagen hydrolysate addition on the growth of strains in the biofilm while the LGG inoculum amount is changed in the multi-species biofilm model.
FIG. 12 is a view showing an experimental procedure for generating a single biofilm model and dual biofilm model.
FIG. 13 is a view confirming the effect of simultaneous treatment of LGG and collagen hydrolysate on biofilm production by *S. mutans* in the single and dual biofilm models. FIG. 3A shows the results of confirming the number of viable cells of *S. mutans* and LGG. FIG. 3B shows the results of confirming the pH. FIG. 3C shows the results of confirming the biofilm dry weight.

### [Modes of the Invention]

Hereinafter, the present invention is described in detail.

The present invention provides a composition for inhibiting biofilm, the composition including at least one selected from the group consisting of *Lactobacillus rhamnosus,* its culture medium, its extract, and its cell-free supernatant; and collagen hydrolysate.

The present inventors studied a method for effectively inhibiting the biofilm produced by *Streptococcus mutans* and confirmed that *Streptococcus mutans* and collagen hydrolyzate are simultaneously treated to effectively inhibit biofilm, thereby completing the present invention.

The *Lactobacillus rhamnosus* is mainly found in yogurt and other dairy products including infant formula and is a bacterium commonly used as a probiotic. The scientific classification of *Lactobacillus rhamnosus* is as follows: Kingdom: Bacteria, Phylum: Firmicutes, Class: Bacilli, Order: Lactobacillales, Family: Lactobacillaceae, Genus: Lactobacillus, Species: Lactobacillus rhamnosus.

In the present invention, the *Lactobacillus rhamnosus* is *Lactobacillus rhamnosus* GG DSM 33156, and specifically, it is a strain deposited as DSM33156 in the DSMZ culture collection.

The *Lactobacillus rhamnosus* may be included in a concentration of 1.0 × 10⁵ to 1.0 × 10¹² colony-forming unit (CFU)/ml, preferably 1.0×10⁶ to 1.0×10¹² CFU/ml, 1.0×10⁷ to 1.0×10¹² CFU/ml, 1.0×10⁸ to 1.0×10¹² CFU/ml, 1.0×10⁹ to 1.0×10¹² CFU/ml, 1.0×10¹⁰ to 1.0×10¹² CFU/ml, 1.0×10¹¹ to 1.0×10¹² CFU/ml, 1.0×10⁵ to 1.0×10¹¹ CFU/ml, 1.0×10⁶ to 1.0×10¹¹ CFU/ml, 1.0×10⁷ to 1.0×10¹¹ CFU/ml, 1.0×10⁸ to 1.0×1011 CFU/ml, 1.0×10⁹ to 1.0×10¹¹ CFU/ml, 1.0×10¹⁰ to 1.0×10¹¹ CFU/ml, 1.0×10⁵ to 1.0×10¹⁰ CFU/ml, 1.0×10⁶ to 1.0×10¹⁰ CFU/ml, 1.0×10⁷ to 1.0×10¹⁰ CFU/ml, 1.0×10⁸ to 1.0×10¹⁰ CFU/ml, 1.0×10⁹ to 1.0×10¹⁰ CFU/ml, 1.0×10⁵ to 1.0×10⁹ CFU/ml, 1.0×10⁶ to 1.0×10⁹ CFU/ml, 1.0×10⁷ to 1.0×10⁹ CFU/ml, 1.0×10⁸ to 1.0×10⁹ CFU/ml, 1.0×10⁵ to 1.0×10⁸ CFU/ml, 1.0×10⁶ to 1.0×10⁸ CFU/ml, 1.0×10⁷ to 1.0×10⁸ CFU/ml, 1.0×10⁵ to 1.0×10⁷ CFU/ml, 1.0×10⁶ to 1.0×10⁷ CFU/ml or 1.0×10⁵ to 1.0×10⁶ CFU/ml in the composition of the present invention.

In the above concentration, it may be more preferably included in a concentration of 1.0×10⁷ to 1.0×10¹² CFU/ml, and even more preferably may be included in a concentration of 1.0×10⁸ CFU/ml.

As used herein, the term "culture solution" includes, without limitation, the culture solution itself culturing the strain in a suitable medium, the filtrate from which the strain is removed by filtering or centrifuging the culture solution (a liquid that has passed through a filter or centrifuged supernatant), the concentrated solution of the filtrate, cell lysate obtained by sonicating culture solution or treating the culture solution with lysozyme, a product obtained after culturing the strain, etc.. The composition of the culture solution may additionally include a component that acts synergistically on the growth of *Lactobacillus rhamnosus* in addition to a component required for conventional *Lactobacillus rhamnosus* culture. The composition thereof can be easily selected by those of ordinary skill in the art.

As used herein, the term "cell-free supernatant" refers to a pure liquid obtained by culturing a strain, centrifuging it, and removing the filtered cells using a filter. The cell-free supernatant refers to a liquid filtered using a filter after culturing the strain and centrifuging it. The composition of the present invention may include, without limitation, the *Lactobacillus rhamnosus* strain, the culture solution of the strain, the cell-free supernatant of the strain, its concentrate, its extract, or its dried product, in which the drying method may include ventilation drying, natural drying, spray drying and lyophilization, but not limited thereto.

As used herein, the term "collagen" is a kind of tissue-forming protein that fills the extracellular space of connective tissue, also called elastin. The collagen hydrolysate of the present invention may be a material obtained by hydrolyzing collagen obtained from any one or more individuals selected from the group consisting of porcine, bovine, poultry, and fish and shellfish, but is not limited thereto.

More preferably, the collagen hydrolysate is obtained by isolating a tissue of an individual with an enzyme and may have a molecular weight of 150 to 8000 Da, preferably 150 to 5000 Da, but is not limited thereto.. As the enzyme, any known enzyme capable of degrading proteins may be used without limitation. In one embodiment of the present invention, collagen hydrolysate derived from pig skin having a pH of 5.0 to 6.5 and an average molecular weight of about 3000 Da was used.The collagen hydrolysate is characterized in that the G-X-Y structure is repeated. G means glycine, and X and Y mean other amino acids. Proline or hydroxyproline is mainly located, but other amino acids may be located. The collagen hydrolysate of the present invention may be a tri-peptide having a G-X-Y structure, and a dipeptide having a G-X structure, a fragmented peptide structure that may be generated in the process of enzymatic treatment of the tri-peptide but is not limited thereto.

Further, the collagen hydrolysate may include peptides characterized by Gly and Hyp such as Gly-Hyp, Gly-Pro-Hyp, Pro-Hyp, Pro-Hyp-Gly, Ala-Hyp, Ala-Hyp-Gly, Ser-Hyp-Gly, Ile-Hyp, Leu-Hyp, and Phe-Hyp in addition to Gly-Pro, Gly-Pro-Ala, Gly-Phe-Ala, Pro-Gly-Gly, and Gly-Pro-Val, but are limited thereto (Gly: glycine, Pro: proline, Ala: alanine, Val: valine, Hyp: hydroxyproline, Ser: serine, Ile: isoleucine, Leu: leucine, and Phe: phenylalanine).

The collagen hydrolysate of the present invention is characterized in that it preferably contains 30 to 40% of glycine.

The collagen hydrolysate may be included at a concentration of 1 to 10000 mg/ml, and the concentration may vary depending on the formulation, but is not limited thereto.For example, when the composition of the present invention is provided in a liquid form, it may be included at a concentration of 1 to 20 mg/ml, preferably 1 to 19 mg/ml, 1 to 18 mg/ml, or 1 to 17 mg/ml. ml, 1 to 16 mg/ml, 1 to 15 mg/ml, 1 to 14 mg/ml, 1 to 13 mg/ml, 1 to 12 mg/ml, 1 to 11 mg/ml, 1 to 10 mg/ml , 1 to 9 mg/ml, 1 to 8 mg/ml, 1 to 7 mg/ml, 1 to 6 mg/ml, 1 to 5 mg/ml, 1 to 4 mg/ml, 1 to 3 mg/ml, 1 to 2 mg/ml, 2 to 20 mg/ml, 2 to 19 mg/ml, 2 to 18 mg/ml, 2 to 17 mg/ml, 2 to 16 mg/ml, 2 to 15 mg/ml, 2 to 14 mg/ml, 2 to 13 mg/ml, 2 to 12 mg/ml, 2 to 11 mg/ml, 2 to 10 mg/ml, 2 to 9 mg/ml, 2 to 8 mg/ml, 2 to 7 mg/ml, 2 to 6 mg/ml, 2 to 5 mg/ml, 2 to 4 mg/ml, 2 to 3 mg/ml, 3 to 20 mg/ml, 3 to 19 mg/ml, 3 to 18 mg/ml, 3 to 17 mg/ml, 3 to 16 mg/ml, 3 to 15 mg/ml, 3 to 14 mg/ml, 3 to 13 mg/ml, 3 to 12 mg/ml, 3 to 11 mg /ml, 3 to 10 mg/ml, 3 to 9 mg/ml, 3 to 8 mg/ml, 3 to 7 mg/ml, 3 to 6 mg/ml, 3 to 5 mg/ml, 3 to 4 mg/ ml, 4 to 20 mg/ml, 4 to 19 mg/ml, 4 to 18 mg/ml, 4 to 17 mg/ml, 4 to 16 mg/ml, 4 to 15 mg/ml, 4 to 14 mg/ml , 4 to 13 mg/ml, 4 to 12 mg/ml, 4 to 11 mg/ml, 4 to 10 mg/ml, 4 to 9 mg/ml, 4 to 8 mg/ml, 4 to 7 mg/ml, 4 to 6 mg/ml, 4 to 5 mg/ml, 5 to 20 mg/ml, 5 to 19 mg/ml, 5 to 18 mg/ml, 5 to 17 mg/ml, 5 to 16 mg/ml, 5 to 15 mg/ml, 5 to 14 mg/ml, 5 to 13 mg/ml, 5 to 12 mg/ml, 5 to 11 mg/ml, 5 to 10 mg/ml, 5 to 9 mg/ml, 5 to 8 mg/ml, 5 to 7 mg/ml, 5 to 6 mg/ml, 6 to 20 mg/ml, 6 to 19 mg/ml, 6 to 18 mg/ml, 6 to 17 mg/ml, 6 to 16 mg/ml, 6 to 15 mg/ml, 6 to 14 mg/ml,6 to 13 mg/ml, 6 to 12 mg/ml, 6 to 11 mg/ml, 6 to 10 mg/ml, 6 to 9 mg /ml, 6 to 8 mg/ml, 6 to 7 mg/ml, 7 to 20 mg/ml, 7 to 19 mg/ml, 7 to 18 mg/ml, 7 to 17 mg/ml, 7 to 16 mg/ ml, 7 to 15 mg/ml, 7 to 14 mg/ml, 7 to 13 mg/ml, 7 to 12 mg/ml, 7 to 11 mg/ml, 7 to 10 mg/ml, 7 to 9 mg/ml , 7 to 8 mg/ml, 8 to 20 mg/ml, 8 to 19 mg/ml, 8 to 18 mg/ml, 8 to 17 mg/ml, 8 to 16 mg/ml, 8 to 15 mg/ml, 8 to 14 mg/ml, 8 to 13 mg/ml, 8 to 12 mg/ml, 8 to 11 mg/ml, 8 to 10 mg/ml, 8 to 9 mg/ml, 9 to 20 mg/ml, 9 to 19 mg/ml, 9 to 18 mg/ml, 9 to 17 mg/ml, 9 to 16 mg/ml, 9 to 15 mg/ml, 9 to 14 mg/ml, 9 to 13 mg/ml, 9 to 12 mg/ml, 9 to 11 mg/ml, 9 to 10 mg/ml, 10 to 20 mg/ml, 10 to 19 mg/ml, 10 to 18 mg/ml, 10 to 17 mg/ml, 10 to 16 mg/ml, 10 to 15 mg/ml, 10 to 14 mg/ml, 4 to 13 mg/ml, 10 to 12 mg/ml, 10 to 11 mg/ml, 11 to 20 mg/ml, 11 to 19 mg /ml, 11 to 18 mg/ml, 11 to 17 mg/ml, 11 to 16 mg/ml, 11 to 15 mg/ml, 11 to 14 mg/ml, 11 to 13 mg/ml, 11 to 12 mg/ ml, 12 to 20 mg/ml, 12 to 19 mg/ml, 12 to 18 mg/ml, 12 to 17 mg/ml, 12 to 16 mg/ml, 12 to 15 mg/ml, 12 to 14 mg/ml, 12 to 13 mg/ml, 13 to 20 mg/ml, 13 to 19 mg/ml, 13 to 18 mg/ml, 13 to 17 mg/ml, 13 to 16 mg/ml, 13 to 15 mg/ml, 13 to 14 mg/ml, 14 to 20 mg/ml, 14 to 19 mg/ml, 14 to 18 mg/ml, 14 to 17 mg/ml, 14 to 16 mg/ml, 14 to 15 mg/ml, 15 to 20 mg/ml, 15 to 19 mg/ml, 15 to 18 mg/ml, 15 to 17 mg/ml, 15 to 16 mg/ml, 16 to 20 mg/ml, 16 to 19 mg/ml, 16 to 18 mg/ml, 16 to 17 mg/ml, 17 to 20 mg/ml, 17 to 19 mg/ml, 17 to 18 mg/ml, 18 to 20 mg/ml, 18 to 19 mg/ml or 19 to 20.

In the above concentration, it may be included at a concentration of 2 to 10 mg/ml, and even more preferably at a concentration of 5 mg/ml.

Further, when the composition of the present invention is provided in powder form, it may be included in a concentration of 100 to 2000 mg/ml, preferably 100 to 1900 mg/ml, 100 to 1800 mg/ml, 100 to 1700 mg/ml, 100 mg/ml. to 1600 mg/ml, 100 to 1500 mg/ml, 100 to 1400 mg/ml, 100 to 1300 mg/ml, 100 to 1200 mg/ml, 100 to 1100 mg/ml, 100 to 1000 mg/ml, 100 to 1000 mg/ml 900 mg/ml, 100 to 800 mg/ml, 100 to 700 mg/ml, 100 to 600 mg/ml, 100 to 500 mg/ml, 100 to 400 mg/ml, 100 to 300 mg/ml, 100 to 200 mg/ml, 200 to 2000 mg/ml, 200 to 1900 mg/ml, 200 to 1800 mg/ml, 200 to 1700 mg/ml, 200 to 1600 mg/ml, 200 to 1500 mg/ml, 200 to 1400 mg /ml, 200 to 1300 mg/ml, 200 to 1200 mg/ml, 200 to 1100 mg/ml, 200 to 1000 mg/ml, 200 to 900 mg/ml, 200 to 800 mg/ml, 200 to 700 mg/ ml, 200 to 600 mg/ml, 200 to 500 mg/ml, 200 to 400 mg/ml, 200 to 300 mg/ml, 300 to 2000 mg/ml, 300 to 1900 mg/ml, 300 to 1800 mg/ml, 300 to 1700 mg/ml, 300 to 1600 mg/ml, 300 to 1500 mg/ml, 300 to 1400 mg/ml, 300 to 1300 mg/ml, 300 to 1200 mg/ml, 300 to 1100 mg/ml, 300 to 1000 mg/ml, 300 to 900 mg/ml, 300 to 800 mg/ml, 300 to 700 mg/ml, 300 to 600 mg/ml, 300 to 500 mg/ml, 300 to 400 mg/ml, 400 to 2000 mg/ml, 400 to 1900 mg/ml, 400 to 1800 mg/ml, 400 to 1700 mg/ml, 400 to 1600 mg/ml, 400 to 1500 mg/ml, 400 to 1400 mg/ml, 400 to 1300 mg/ml, 400 to 1200 mg/ml, 400 to 1100 mg/ml, 400 to 1000 mg/ml, 400 to 900 mg/ml, 400 to 800 mg/ml, 400 to 700 mg/ml, 400 to 600 mg/ml, 400 to 500 mg/ml, 500 to 2000 mg/ml, 500 to 1900 mg/ml, 500 to 1800 mg/ml, 500 to 1700 mg/ml, 500 to 1600 mg/ml, 500 to 1500 mg /ml, 500 to 1400 mg/ml, 500 to 1300 mg/ml, 500 to 1200 mg/ml, 500 to 1100 mg/ml, 500 to 1000 mg/ml, 500 to 900 mg/ml, 500 to 800 mg/ ml, 500 to 700 mg/ml, 500 to 600 mg/ml, 600 to 2000 mg/ml, 700 to 1900 mg/ml, 700 to 1800 mg/ml, 700 to 1700 mg/ml, 700 to 1600 mg/ml , 700 to 1500 mg/ml, 700 to 1400 mg/ml, 700 to 1300 mg/ml, 700 to 1200 mg/ml, 700 to 1100 mg/ml, 700 to 1000 mg/ml, 700 to 900 mg/ml, 700 to 800 mg/ml, 800 to 2000 mg/ml, 800 to 1900 mg/ml, 800 to 1800 mg/ml, 800 to 1700 mg/ml, 800 to 1600 mg/ml, 800 to 1500 mg/ml, 800 to 1400 mg/ml, 800 to 1300 mg/ml, 800 to 1200 mg/ml, 800 to 1100 mg/ml, 800 to 1000 mg/ml, 800 to 900 mg/ml, 900 to 2000 mg/ml, 900 to 900 mg/ml 1900 mg/ml, 900 to 1800 mg/ml, 900 to 1700 mg/ml, 900 to 1600 mg/ml, 900 to 1500 mg/ml, 900 to 1400 mg/ml, 900 to 1300 mg/ml, 900 to 1200 mg/ml, 900 to 1100 mg/ml, 900 to 1000 mg/ml, 1000 to 2000 mg/ml, 1000 to 1900 mg/ml, 1000 to 1800 mg/ml, 1000 to 1700 mg/ml, 1000 to 1600 mg /ml, 1000 to 1500 mg/ml, 1000 to 1400 mg/ml, 1000 to 1300 mg/ml, 1000 to 1200 mg/ml, 1000 to 1100 mg/ml, 1200 to 2000 mg/ml, 1200 to 1900 mg/ ml, 1200 to 1800 mg/ml, 1200 to 1700 mg/ml, 1200 to 1600 mg/ml, 1200 to 1500 mg/ml, 1200 to 1400 mg/ml, 1200 to 1300 mg/ml, 1300 to 2000 mg/ml , 1300 to 1900 mg/ml, 1300 to 1800 mg/ml, 1300 to 1700 mg/ml, 1300 to 1600 mg/ml, 1300 to 1500 mg/ml, 1300 to 1400 mg/ml, 1400 to 2000 mg/ml, 1400 to 1900 mg/ml, 1400 to 1800 mg/ml, 1400 to 1700 mg/ml, 1400 to 1600 mg/ml, 1400 to 1500 mg/ml, 1500 to 2000 mg/ml, 1500 to 1900 mg/ml, 1500 to 1800 mg/ml, 1500 to 1700 mg/ml, 1500 to 1600 mg/ml, 1500 to 1500 mg/ml, 1600 to 2000 mg/ml, 1600 to 1900 mg/ml, 1600 to 1800 mg/ml, 1600 to 1600 mg/ml It may be included in a concentration of 1700 mg/ml, 1700 to 2000 mg/ml, 1700 to 1900 mg/ml, 1700 to 1800 mg/ml, 1800 to 2000 mg/ml, 1800 to 1900 mg/ml or 1900 to 2000 mg/ml.

In the above concentration, it may be included at a concentration of 200 to 1000 mg/ml, more preferably 500 mg/ml.

The composition of the present invention is characterized in that least one selected from the group consisting of *Lactobacillus rhamnosus,* its culture medium, its extract and its cell-free supernatant and collagen hydrolysate are contained and mixed at each optimal concentration.

According to an embodiment of the present invention, the collagen hydrolyzate showed an effect of inhibiting acid production in *Lactobacillus rhamnosus* or *Streptococcus mutans* strains.

Further, according to another embodiment of the present invention, the simultaneous treatment of *Lactobacillus rhamnosus* and collagen hydrolysate could significantly inhibit the biofilm produced by *Streptococcus mutans* than *Lactobacillus rhamnosus* alone treatment. Thus, it is confirmed that the biofilm inhibitory ability is increased by the simultaneous treatment. Biofilm inhibition of the present invention is used to include not only the inhibition of biofilm formation, but also the effect of suppressing and reducing the generated biofilm.

In addition, according to another embodiment of the present invention, the simultaneous treatment of the *Lactobacillus rhamnosus* and the collagen hydrolyzate does not entail sterilization against *Streptococcus mutans* and promotes the growth of *Streptococcus oralis* (*S. oralis*) in the biofilm when the *Lactobacillus rhamnosus* is treated at a concentration of 1.0 × 10⁷ CFU/ml.

The *Streptococcus oralis* (previously known as *S. sanguis* type II) is known to play an important role in maintaining the normal healthy shape of microorganisms including periodontal flora.

The *S. oralis* can be found in plaque, and in particular, it has been proven to contribute to periodontal health by inhibiting colony formation of periodontal pathogens such as *Aggregatibacter actinomycetemcomitans, Porphyromonas gingivalis, Peptostreptococcus micros* and *Campylobacter rectus.*

Therefore, simultaneous treatment of *Lactobacillus rhamnosus* and collagen hydrolysate of the present invention may further help oral health by inhibiting biofilm production by *S. mutans* and promoting the growth of *S. oralis.*

The present invention provides an oral composition for inhibiting biofilm formation including at least one selected from the group consisting of *Lactobacillus rhamnosus,* its culture medium, its extract and its cell-free supernatant and collagen hydrolysate.

In addition, in order to achieve the above other object, the present invention provides a method for inhibiting biofilm comprising the step of administering to an individual at least one selected from the group consisting of *Lactobacillus rhamnosus,* its culture medium, its extract and its cell-free supernatant; and collagen hydrolysate. The oral composition may be used for maintaining and improving oral health, such as caries suppression and stomatitis prevention effect based on the inhibitory ability of biofilm formation by treating *S. mutans* of *Lactobacillus rhamnosus* and collagen hydrolysate at the same time.

The oral composition may be provided in all types and formulations that can be used for oral hygiene. Examples thereof include toothpaste, mouthwash, gum, oral spray, oral gel, oral ointment, and oral patch but are not limited thereto. Further, the oral composition may be applied to oral hygiene products such as toothbrushes, dental floss, interdental toothbrushes, tongue cleaners, and oral wipes.

When the oral composition of the present invention is used for toothpaste, it may further include abrasives, binders, humectants, foaming agents, sweeteners, whitening agents, or flavoring agents in addition to at least one selected from the group consisting of *Lactobacillus rhamnosus,* its culture medium, its extract and its cell-free supernatant and collagen hydrolysate as active ingredients.

Examples of the abrasive include aluminum hydroxide, silicic anhydride, aluminum silicate, dicalcium phosphate dihydrate and anhydride, tricalcium phosphate, calcium carbonate, calcium pyrophosphate, insoluble sodium metaphosphate, magnesium triphosphate, magnesium carbonate, calcium sulfate, polymethyl methacrylate, etc. alone or in combination. The content of the abrasive may usually be 20% by weight to 90% by weight based on the total composition but is not limited thereto. When the oral hygiene composition is a paste composition, organic binders including various cellulose derivatives for thickening such as carrageenan, gums such as xanthan gum and tragacanth gum, synthetic polymer derivatives such as polyvinyl alcohol, sodium polyacrylate, polyacrylic acid/ maleic acid copolymer and carboxyvinyl polymer and inorganic binders including silica and laponite can be used alone or in combination. The content of the binder may usually be 0.3% by weight to 5% by weight based on the total composition but is not limited thereto.

In addition, the oral composition may further include sorbitol, glycerin, ethylene glycol, propylene glycol, polyether glycol, polypropylene glycol, and the like as a moisturizing agent during the preparation thereof.

In addition, the oral composition may further include a flavoring agent or a sweetener. The sweetener may be sucrose, lactose, maltose, sorbitol, xylitol, sodium cyclamate, glycerin, sodium saccharin, stevioside, aspartame, and the like, and the flavoring agent may be peppermint, menthol, anethole, eugenol, limonene, citronellol, alpha-terpineol, methyl salicylate, cineol, linalool, ethylinalool, vanillin, thymol, spearmint oil, seji oil, rosemary oil, cinnamon oil, etc., and the sweetener or flavoring agent may be used alone or in combination.

Further, the oral composition of the present invention may include a surfactant or additional effective component used as a foaming component alone or in combination, and the surfactant used as the foaming component may be any one selected from the group consisting of an anionic surfactant, a nonionic surfactant and a cationic surfactant. More specifically, sodium lauryl sulfate as an anionic surfactant and polyoxyethylene polyoxypropylene copolymer (poloxamer), polyoxyethylene hardened castor oil, polyoxyethylene sorbitan fatty acid ester, etc. as a nonionic surfactant, may be used without limitation.

When the oral composition of the present invention is used for toothpaste, it can be prepared by the conventional method of preparing toothpaste except for containing at least one selected from the group consisting of *Lactobacillus rhamnosus,* its culture medium, its extract, and its cell-free supernatant and collagen hydrolysate as active ingredients. When the oral composition of the present invention is used for a toothbrushing solution, at least one selected from the group consisting of *Lactobacillus rhamnosus,* its culture medium, its extract, and its cell-free supernatant and collagen hydrolysate as active ingredients may be mixed to be formulated into toothbrushing solution. Oral diseases may be prevented, alleviated, or treated by washing the oral cavity 2 to 10 times a day.

Further, when the oral composition of the present invention is used for a mouthwash (cleansing agent), it may further include a toothpaste carrier, more specifically, non-toxic alcohol. In the present invention, at least one selected from the group consisting of *Lactobacillus rhamnosus,* its culture medium, its extract and its cell-free supernatant and collagen hydrolysate may be included in an amount of 0.00001 to 10% by weight relative to the total weight of the composition, preferably in an amount of 0.0005 to 5% by weight relative to the total weight of the composition, and more preferably in an amount of 0.005 to 1% by weight relative to the total weight of the composition, but is not limited thereto.

Further, the present invention provides a pharmaceutical composition for preventing or treating oral diseases caused by *Streptococcus mutans,* the composition including at least one selected from the group consisting of *Lactobacillus rhamnosus,* its culture medium, its extract, and its cell-free supernatant; and collagen hydrolysate.

In addition, in order to achieve the above further object, the present invention provides a method for treating globular disease caused by *Streptococcus mutans* comprises the step of administering to an individual at least one selected from the group consisting of *Lactobacillus rhamnoses,* its culture medium, its extract and its cell-free supernatant; and collagen hydrolysate. The oral disease may include at least one selected from the group consisting of dental caries, periodontitis, stomatitis, gum recession, gum hypertrophy and gingivitis but is not limited thereto.

As used herein, the term "prevention" may refer to any action of inhibiting or delaying the onset of oral disease by administering the composition for preventing or treating oral disease according to the present invention to an individual.

As used herein, the term "treatment" is an approach for obtaining beneficial or desirable clinical results. Regardless of whether it is detectable or impossible partial or total, beneficial, or desirable clinical results for the purposes of the present invention, it may include alleviation of symptoms, reduction of the severity of the disease, a stabilized (i.e., not worsening) state of the disease, delaying or reducing the rate of disease progression, amelioration or temporary alleviation and reduction of the disease state, but is not limited thereto. Accordingly, treatment refers to both therapeutic treatment and prophylactic levels, and those that need to be treated include those already having the disease as well as those in which the disease is to be prevented. In addition, it may refer to any action to improve or benefit the symptoms of oral disease by administering the pharmaceutical composition of the present invention to an individual.

As used herein, the term "individual" may refer to any animal, including humans, that has or is likely to develop an oral disease.

The pharmaceutical composition of the present invention may be formulated and used in the form of oral dosage forms such as powders, granules, tablets, capsules, suspensions, emulsions, syrups, aerosols, external preparations, suppositories, and sterile injection solutions, respectively, according to conventional methods. Carriers, excipients, and diluents that may be included in the pharmaceutical composition may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate and mineral oil. In the case of formulation, diluents, or excipients, which are commonly used, such as fillers, extenders, binders, wetting agents, disintegrants, and surfactants are used and prepared. Solid preparations for oral administration include tablets, pills, powders, granules, capsules, etc. Such solid preparations include at least one excipient, for example, starch, calcium carbonate, sucrose or lactose, gelatin, etc. in addition to a mixture of at least one selected from the group consisting of *Lactobacillus rhamnosus* of the present invention, its culture solution, its extract, and its cell-free supernatant and collagen hydrolysate. In addition to simple excipients, lubricants such as magnesium stearate and talc are also used. Liquid formulations for oral use include suspensions, solutions, emulsions, syrups, etc. In addition to water and liquid paraffin, which are commonly used simple diluents, various excipients such as wetting agents, sweeteners, fragrances, and preservatives may be included. Formulations for parenteral administration include sterile aqueous solutions, non-aqueous solutions, suspensions, emulsions, freeze-dried preparations, and suppositories. Non-aqueous solutions and suspensions include propylene glycol, polyethylene glycol, vegetable oils such as olive oil, and injectable esters such as ethyl oleate. As the suppository base, witepsol, macrogol, tween 61, cacao butter, laurin, glycerogelatin, and the like may be used.

The dosage of the pharmaceutical composition of the present invention varies depending on the age, sex, and weight of the individual to be treated, the specific disease or pathological condition to be treated, the severity of the disease or pathological condition, the route of administration, and the judgment of the prescriber. Dosage determination based on these factors is within the level of one of ordinary skill in the art, and generally dosages range from 0.01 mg/kg/day to approximately 2,000 mg/kg/day. A more preferred dosage is 0.1 mg/kg/day to 1,000 mg/kg/day. Administration may be administered once a day or may be administered in several divided doses. The above dosage does not limit the scope of the present invention in any way.

The pharmaceutical composition of the present invention may be administered to mammals such as rats, livestock, and humans by various routes and may be used parenterally or orally, but preferably may be administered parenterally.

Forms for parenteral administration include toothpaste, mouthwash, and topical administration agents (creams, ointments, dressing solutions, sprays, other coating agents, etc.). As an example of the formulation of the topical administration agent, the pharmaceutical composition for preventing or treating oral diseases according to the present invention is fixed to a film or patch containing a water-soluble polymer, and it may be formulated so as to attach to teeth and surrounding areas.

In the present invention, the pharmaceutical composition for preventing or treating oral disease, in addition to the active ingredient, may further include any compound or natural extract known to have an oral disease therapeutic effect and has already been tested for safety in order to increase the therapeutic effect of oral diseases.

Further, the pharmaceutical composition for preventing or treating oral diseases of the present invention may be used together with surgical treatment of oral diseases.

Further, the present invention provides a food composition for preventing or alleviating oral diseases caused by *Streptococcus mutans,* the composition including at least one selected from the group consisting of *Lactobacillus rhamnosus,* its culture medium, its extract and its cell-free supernatant; and collagen hydrolysate.

The food composition according to the present invention includes all types of functional food, nutritional supplement, health food, health functional food and food additives.

As used herein, the term "health functional food" refers to food manufactured and processed by extracting, concentrating, refining, mixing, etc., a specific ingredient as a raw material or a specific ingredient in a food raw material for the purpose of health supplementation and refers to food designed and processed to sufficiently exert biological control functions such as biological defense, regulation of biological rhythm, prevention and recovery of disease, etc., by the above ingredients, and performs functions related to prevention of disease or recovery of health, etc.

The food composition according to the present invention can be prepared in various forms according to conventional methods known in the art.

For example, as a health food, a mixture itself of at least one selected from the group consisting *of Lactobacillus rhamnosus* of the present invention, its culture medium, its extract and its cell-free supernatant and collagen hydrolysate may be granulated, encapsulated, and powdered. It can be prepared and ingested in the form of teas, juices, and drinks. Further, the mixture of at least one selected from the group consisting of *Lactobacillus rhamnosus* of the present invention, its culture medium, its extract and its cell-free supernatant and collagen hydrolysate may be prepared in the form of a composition by mixing with known substances or active ingredients having a biofilm formation inhibitory effect or oral disease prevention, improvement or treatment effect.

Further, functional foods may be prepared by adding beverages (including alcoholic beverages), fruits and their processed foods (e.g., canned fruit, bottled food, jam, marmalade, etc.), fish, meat, and their processed foods (e.g., ham, sausage corn beef, etc.), breads and noodles (e.g., udon noodles, soba noodles, ramen, spaghetti, macaroni, etc.), fruit juice, various drinks, cookies, syrup, dairy products (e.g., butter, cheese, etc.), edible vegetable oil, margarine, vegetable protein, retort food, frozen food, various seasonings (e.g., soybean paste, soy sauce, sauce, etc.) to a mixture of at least one selected from the group consisting of *Lactobacillus rhamnosus* of the present invention, its culture medium, its extract and its cell-free supernatant and collagen hydrolysate.

In the food composition of the present invention, the preferred content of a mixture of at least one selected from the group consisting of *Lactobacillus rhamnosus* of the present invention, its culture medium, its extract, and its cell-free supernatant and collagen hydrolysate may be, for example, 0.01 to 80% by weight, preferably 0.01 to 50% by weight of the finally prepared food but is not limited thereto.

Further, in order to use the food composition of the present invention in the form of a food additive, it may be prepared and used in the form of powders or concentrates.

Further, the present invention provides a feed additive composition for inhibiting biofilm production, the composition including at least one selected from the group consisting of *Lactobacillus rhamnosus,* its culture medium, its extract, and its cell-free supernatant; and collagen hydrolysate.

As used herein, the term "feed" means any natural or artificial food, one meal, or a component of the one meal that the animal eats. The feed additive composition for inhibiting biofilm production according to the present invention as an active ingredient can be prepared in various forms of feed known in the art, and preferably, concentrated feed, roughage and/or special feed may be included, but is not limited thereto.

As used herein, the term "feed additive composition" may be "feed additive" and includes a substance added to feed for the purpose of various effects such as alleviating a symptom of diseases for an animal, supplementing nutrients, and preventing weight loss, improving the digestibility of fiber in feed, improving oil quality, preventing reproductive disorder and improving conception rate, and preventing high temperature stress in summer.

The feed additive composition of the present invention corresponds to the supplementary feed under Control Of Livestock And Fish Feed Act, and may further include mineral preparations such as sodium hydrogen carbonate, bentonite, magnesium oxide, and composite minerals; mineral preparations that are trace minerals such as zinc, copper, cobalt, and selenium; vitamin preparations such as carotene, vitamin A D, E, nicotinic acid, and vitamin B complex; protective amino acid agents such as methionine and lysine; protective fatty acids such as fatty acid calcium salts; live bacteria or yeast such as probiotics (lactic acid bacteria), yeast cultures, and fungal fermentation products.

Among them, the concentrated feed includes seed fruits containing cereals such as wheat, oats, and corn, bran containing rice bran, wheat bran, and barley bran as a by-product of refining and obtaining grain, residues such as residual starch the main component of starch residue, which is the rest of starch removed from sweet potato and potatoes and oilcake, a by-product obtained from soybean, rapeseed, sesame, flaxseed, and coco palm, fish soluble, which is a concentrate of fresh liquid obtained from fish meal, fish residue, and fish, animal feed such as meat powder, blood powder, feather meal, skim milk powder, cheese from milk, dried whey, which is the residue from the production of casein from skim milk, yeast, chlorella and seaweed, but is not limited to thereto.

Among them, roughages include raw grass feeds such as wild grass, pasture, and green grass, root vegetables such as turnip for feed, beet for feed, and Luther Bearer, a kind of turnip, Silage, a storage feed that is fermented with lactic acid by filling raw grass, green grass crops, and grain seeds in silos, hay that has been dried with wild grass and pasture, crop straw for breeding, and legume leaves, but are not limited thereto. Special feeds include mineral feeds such as oyster shells and rock salt, urea feeds such as urea or diureid isobutane, a derivative of urea, and feed additives and dietary supplements, which are substances added in a small amount to the blended feed to supplement ingredients that are likely to be insufficient when adding only the natural feedstock or to increase the storage of the feed.

The feed additive composition according to the present invention may be prepared by adding a mixture of at least one selected from the group consisting *of Lactobacillus rhamnosus,* its culture medium, its extract and its cell-free supernatant and collagen hydrolysate in an appropriate effective concentration range according to various feed preparation methods known in the art.

The feed additive according to the present invention can be applied without limitation as long as it is an individual for the purpose of inhibiting the production of biofilm by *Streptococcus mutans.* For example, it can be applied to any individual including non-human animals such as monkeys, dogs, cats, rabbits, guinee pig, rats, mice, cattle, sheep, pigs, goats, etc., birds and fish.

The above-described contents of the present invention are applied equally to each other unless they contradict each other, and those implemented with appropriate modifications by those skilled in the art are also included in the scope of the present invention.

Hereinafter, the present invention is described in detail through examples, but the scope of the present invention is not limited only to the following examples.

### Example 1. Preparation of strains and collagen hydrolysates

### 1-1. Isolation of strains and confirmation of growth curves

The strain was directly isolated from the LGG strain mixture raw material (Probio-Tec LGG Blend-30, manufacturer: Chr. Hansen A/S, origin: Denmark) and the species-specific primer was used to confirm that the isolated strain was *Lactobacillus rhamnosus* GG DSM 33156 (hereinafter referred to as LGG).

The colony PCR was performed to confirm that the strain isolated from the culture medium was LGG. Specifically, colonies were collected with a sterile tip, transferred to a PCR tube containing a PCR mixture (DW, 10 × Buffer, MgCl₂, dNTP, primer), and then treated at 95°C for 5 minutes. Primer information is as follows: Forward: 5'-CGCCCTTAACAGCAGTCTTC-3' (SEQ ID NO: 1), Reverse: 5'-GCCCTCCGTATGCTTAAACC-3' (SEQ ID NO: 2). Then, Taq enzyme (Taq enzyme) and DW were added and mixed, followed by PCR cycles. PCR cycles were run at 95°C for 5 minutes, 94°C for 15 seconds, 50°C for 30 seconds, and 72°C for 11 seconds × 35 cycles. Then, PCR amplification was performed using agarose gel to confirm the results.

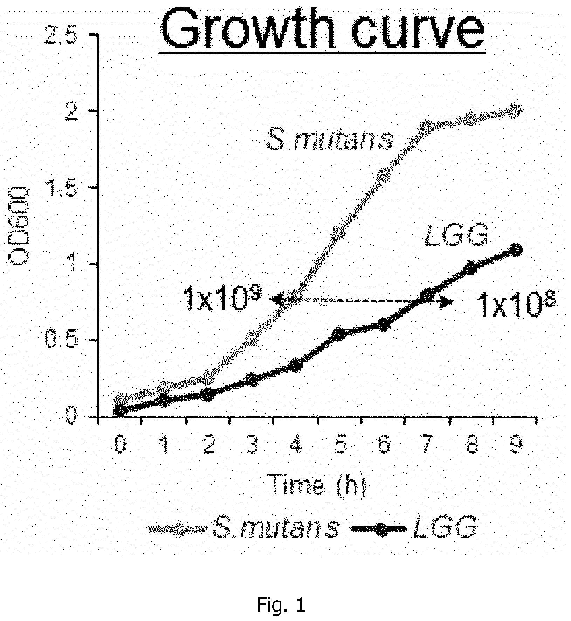

Further, for *in vitro* experiments through culture, ultrafiltered tryptone-yeast (10-kDa cutoff) extract medium (10-kDa molecular mass blocking membrane; Millipore, MA, USA) and 1% glucose was used to confirm the growth curves of LGG and *Streptococcus mutans* (*S. mutans*), a caries pathogen, and the results are shown in FIG. 1.

In order to check the growth curve of the strain, *S. mutans* and LGG activated on agar medium were collected by 2-3 colonies and cultured in ultra-filtered tryptone-yeast extract medium (2.5% tryptone and 1.5% yeast extract/1% (wt/vol) with glucose; pH 7.0) at 37°C and 5% CO₂ for about 18 hours. A certain portion of this culture was re-inoculated into a fresh tryptone-yeast extract medium, and optical density at 600 nm (OD600) was measured every hour using a Spectronic 200 (Thermo, USA).

As shown in FIG. 1, the results indicate that the growth of LGG was somewhat slower than that of *S. mutans,* a caries pathogen, and the CFU showed a difference of about 1-log at a similar OD600 of 0.8. Further, it was confirmed that the growth curves of the two strains cultured in ultra-filtered tryptone-yeast extract medium and BHI (Brain Heart Infusion) medium showed similar trends. Therefore, for the co-culture experiment, it is necessary to separate and confirm the two strains in a specific medium, so a BHI-based blood agar medium was selected as the selective medium.

### 1-2. Preparation of collagen hydrolysate preparation

Collagen hydrolysate derived from pig skin was used as collagen hydrolysate (purchased from PB Liner (USA), product name: SOLUGEL^{®} Prima PP).

### Example 2. Comparison of adhesion between LGG and S. mutans

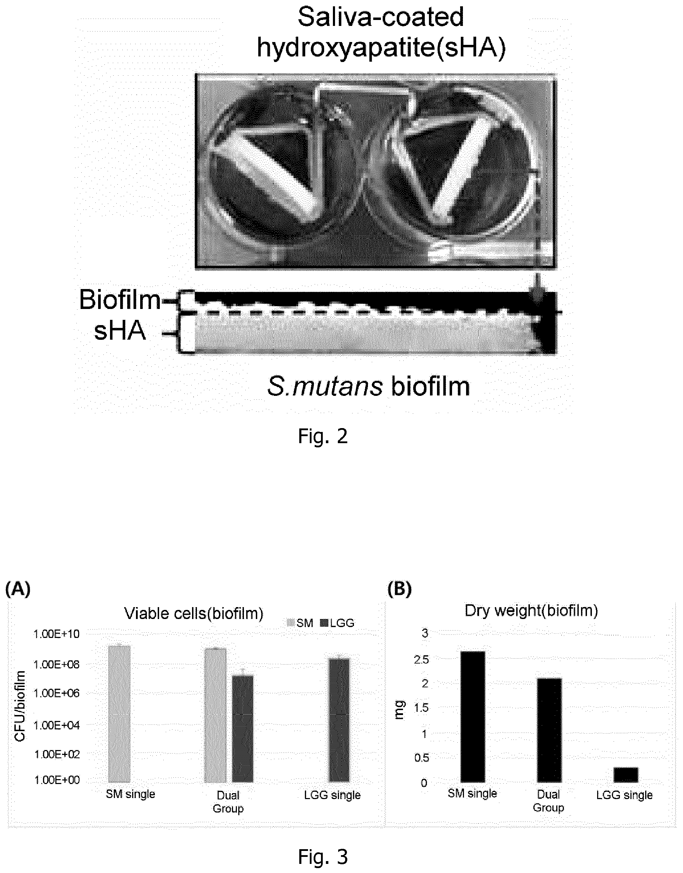

The saliva-coated, hydroxyapatite (HA) disc of FIG. 2 (hereinafter referred to as sHA) was used to compare and verify the adhesion of LGG (inoculum size 10⁵ CFU/ml) and S. *mutans* (inoculum size 10⁵ CFU/ml).

The biofilm was cultured using the saliva-coated sHA disk model in the following method. The sHA disc was placed vertically in a 24-well plate using a self-made disc holder, and they were cultured in 2.8 ml/well ultrafiltration tryptone-yeast extract medium (containing UFTYE (pH 7.0)/1% (wt/vol) sucrose) inoculated with about 10⁵ (CFU/ml) of *S. mutans* (single culture) or 10⁵ (CFU/ml) of LGG at 37°C and 5% CO₂ conditions. For heterogeneous biofilms, *S. mutans* (10⁵ CFU/ml) and LGG (10⁵ CFU/ml) were co-inoculated and cultured.

As described above, the number of viable cells (colony-forming unit (CFU)) and dry weight of each strain on the sHA model were checked to verify the physicochemical/microbiological changes of the biofilm, and the results are shown in FIG. 3.

To check the number of viable cells of each strain, the biofilm of each experimental group was removed from the sHA disk in a sterile 0.89% (w/v) NaCl solution and was homogenized through probe sonication (pulse at 15% amplitude for 30 seconds, output 7W). The homogenized suspension was appropriately diluted and incubated on a blood agar medium at 37°C under the condition of 5% CO₂ for 48 hours. In the case of heterogeneous biofilms, two species were distinguished and counted through colony morphology observation.

To check the dry weight, the homogenized suspension was centrifuged (10000xg, 4°C for 10 minutes) to separate the supernatant from the pellet, and the supernatant was removed. The process of washing with DW and centrifuging the pellet was repeated twice, and then the obtained pellet was freeze-dried to obtain a dried sample. Finally, the dry weight of the freeze-dried sample was measured.

As shown in FIG. 3, it was confirmed that LGG showed 10⁸ CFU/biofilm in the biofilm cultured for 42 hours, which was lower by 1-log than that of *S. mutans* 10⁹ CFU/biofilm, but the ability of adhesion to the sHA surface was excellent. It was determined that the number of viable cells of each strain in the co-culture was not significantly different from the number of viable cells in a single culture.

It was confirmed that the dry weight of *S. mutans,* which well produces *in vitro* polysaccharides, was about 2.6 mg in the case of total biomass, but it was reduced to about 2.1 mg in co-culture. Therefore, it was confirmed that the biofilm production was reduced when LGG and *S. mutans* were co-cultured.

### Example 3. Confirmation of effects of LGG inoculum amount and collagen hydrolysate

The biofilm inhibitory ability was verified by treating the collagen hydrolysate at 5 mg/ml while the inoculum amount of LGG was adjusted to 10⁵, 10⁶ and 10⁷ (CFU/ml). The biofilm inhibitory ability was verified by checking the number of viable cells and dry weight in the same manner as in Example 2.

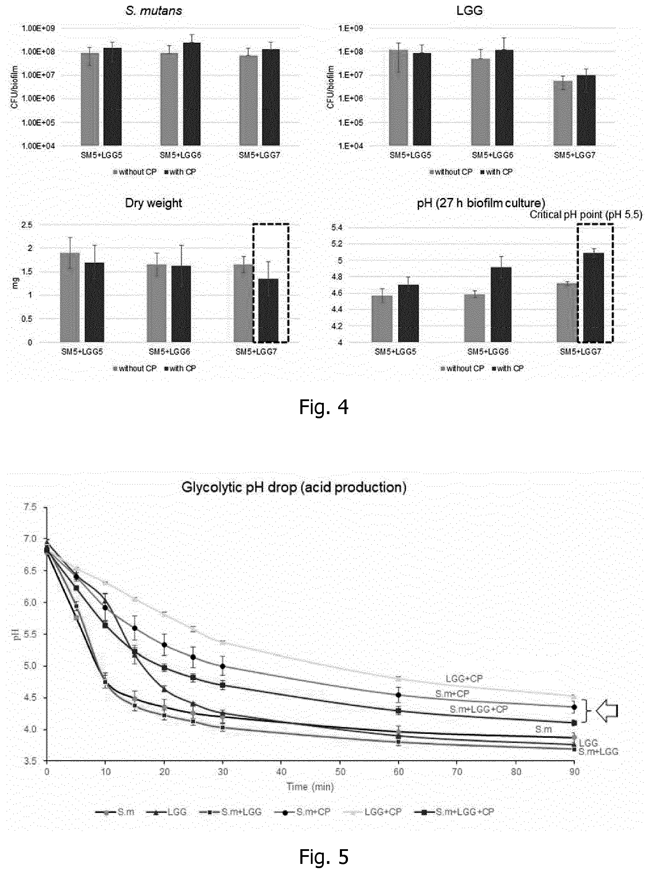

As shown in FIG. 4, the results indicate that *S. mutans* showed similar CFU values regardless of the presence or absence of the LGG viable cell inoculum amount/collagen hydrolysate (CP). The number of LGG viable cells showed a similar trend regardless of the presence or absence of collagen hydrolysate. However,
in the case of inoculation with a large number of viable cells (10⁷), LGG showed a relatively low CFU value compared to other experimental groups. This was judged to be due to the growth of LGG in the late of the biofilm in a state where there was no effect on the initial adhesion. Further, in the case of dry weight, the experimental group treated with the collagen hydrolysate along with LGG at 10⁷ showed relatively low biomass. Further, the treatment of the collagen hydrolysate showed an increased change in the pH value in the culture medium along with the increase in the inoculum amount of the number of LGG viable cells.

Therefore, the above results indicate that collagen hydrolysate significantly affects acid production by glycolysis.

### Example 4. Confirmation of effect of collagen hydrolysate on acid production of S. mutans and LGG

The effect of the collagen hydrolysate on the acid production and acid resistance properties of *S. mutans* and LGG was confirmed through the glycolytic pH drop assay described in the prior art *(See.* International Journal of Oral Science. 2011;3(2):98-106). In the above analysis, the initial pH was set to 6.8 to 7.0. pH was measured every 5 minutes until the initial 30 minutes (acid production rate was checked) and measured every 30 minutes until 90 minutes after 30 minutes (indirectly confirming the acid resistance of the final time). Acid production and acid resistance were measured by treating or not treating the collagen hydrolysate in addition to the single strain *S. mutans* or LGG in the strain mixture.

As shown in FIG. 5, the results indicate that single *S. mutans* (S. m) and S.m + LGG showed a steep pH drop. LGG showed mild for the first 15 minutes but showed a similar level of pH within 30 minutes thereafter. Interestingly, it was shown that treatment with collagen hydrolyzate (CP) controlled the pH drop of S.m or LGG cultures and their mixtures. Therefore, it was determined that the collagen hydrolysate had an effect of controlling acid production by adjusting the glycolysis pathway or affecting F-ATPase.

It was determined that the gentle curve up to the first 30 minutes was due to the influence on acid production through the regulation of the glycolytic pathway, and the higher pH value than the untreated group at the last 90 minutes was due to the result of affecting F-ATPase.

### Example 5. Confirmation of effect according to concentration of collagen hydrolysate

Further, the effect of the collagen hydrolysate was confirmed according to the concentration. The collagen hydrolysate and the control glycine, respectively, were treated by concentration, and the pH was measured in the same manner as in Example 4 (collagen hydrolysate (CP): 0 (control), 2.5, 5, or 10 mg/ml concentration treatment, glycine (Gly): 0 (control), 20 or 40 mM concentration treatment).

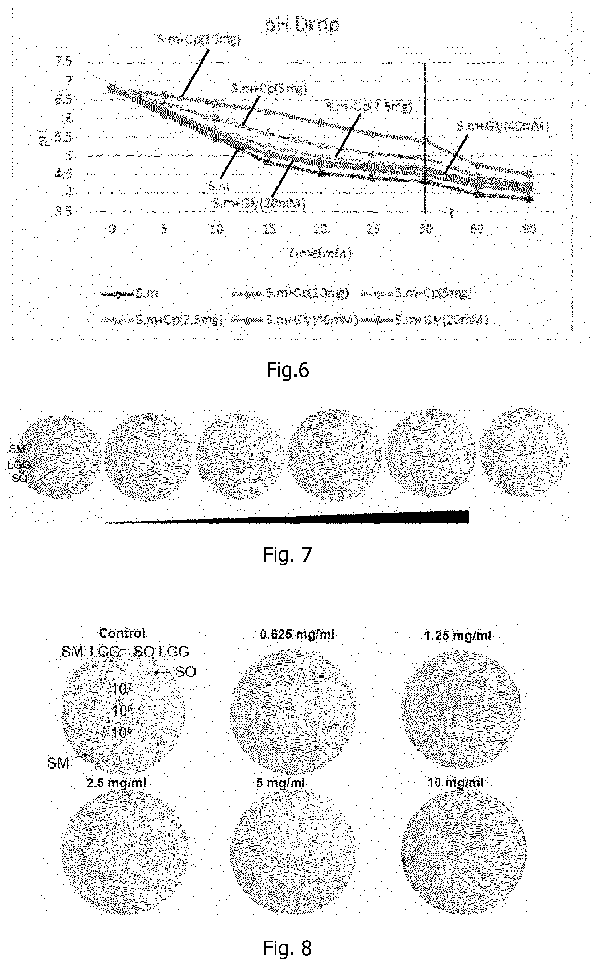

As shown in FIG. 6, the results indicate that the experimental group treated with the collagen hydrolysate showed a clear concentration dependence.

### Example 6. Confirmation of effect of collagen hydrolysate on strain growth

Further, there is a report that glycine contained in the collagen hydrolysate inhibits the growth of gram-negative bacteria. Thus, the effect of collagen hydrolysate (CP) on the growth of S. *mutans* (SM), which is gram-positive bacteria, and a caries bacterium, and its symbiotic bacteria *S. oralis* (SO) and LGG were confirmed.

SM, LGG, and SO were cultured as much as the appropriate number of viable cells (CFU) (1 × 10⁸ CFU/ml), and diluted 10⁻⁵ times in 0.89% NaCl (10⁷, 10⁶, 10⁵, 10⁴, 10³). Then, an agar medium containing the collagen hydrolysate at 0, 0.625, 1.25, 2.5, 5 or 10 mg/ml (final concentration) was used, and 5 µl of bacteria diluted up to 10⁻⁵ times were spotted. Then, they were cultured for 24 hours or 48 hours at 37°C. The spots formed on the non-additive agar medium were compared to the spots formed on the agar medium containing the collagen hydrolysate. It was visually confirmed whether the growth increased or decreased compared to the control. When spots with a high dilution ratio were not formed in the agar medium compared to the control group, it was determined that growth was inhibited.

As shown in FIG. 7, the results indicate that the collagen hydrolysate containing glycine in a high composition ratio did not affect the growth of gram-positive bacteria SM, SO and LGG.

### Example 7. Verification of competitive relationship of each strain according to treatment of collagen hydrolysate

The competitive relationship between pathogens and symbiotic bacteria affects the production of caries-inducing biofilms. Thus, a competition assay was performed to confirm the effects of LGG on the growth of the pathogen *S. mutans* and the symbiotic *S. oralis* depending on the presence or absence of collagen hydrolysate.

The competition between *S. mutans* 10⁵ (CFU/ml) and S. *oralis* 10⁵ (CFU/ml) was verified according to change of LGG inoculum amount 10⁵⁻⁷ (CFU/ml) on agar medium containing collagen hydrolysate at each concentration (0-10 mg/ml final concentration). First, 5 µl of *S. mutans* 10⁵ (CFU/ml) or 5 µl of *S. oralis* 10⁵ (CFU/ml) was inoculated on agar medium, and at the same time, LGG 10⁵⁻⁷ (CFU/ml) was inoculated next to it, and then they were cultured under the condition of 37°C and 5% CO₂ for 48 hours. Then, it was determined that the suppression of specific bacterial spots was determined as competition (*See* Journal of Bacteriology. 2005; 187(21): 7193-7203).

As shown in FIG. 8, the results indicate that when the collagen hydrolysate was used at 0 to 10 mg/ml, there was no competition between *S. mutans* (10⁵) vs. LGG (10⁵⁻⁷) or S. *oralis* (10⁵) vs. LGG (10⁵⁻⁷).

### Example 8. Confirmation of changes in S. mutans and S. oralis populations with or without the addition of LGG inoculum amount or collagen hydrolysate

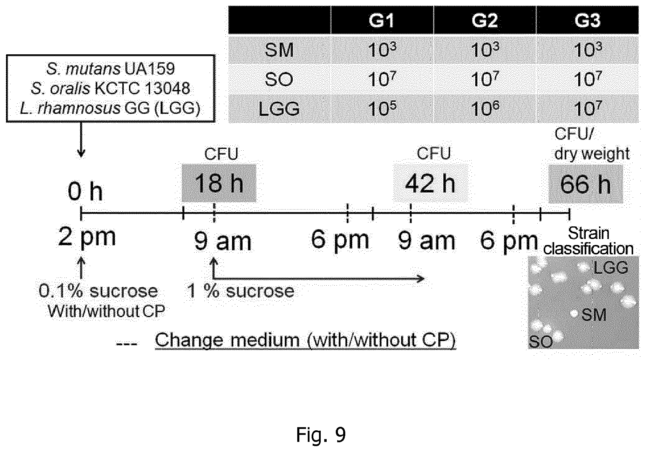

Further, the multi-species biofilm model derived through the process shown in FIG. 9 was used to confirm changes in the *S. mutans* and *S. oralis* populations according to the LGG inoculum amount.

The multi-species biofilm model used an ecological concept. *S. mutans* (UA159), *S.oralis* (KCTC 13048) and LGG were mixed, and collagen hydrolysate (CP) was added on unadded in a medium containing 0.1% sucrose in the initial attachment step. 1% sucrose was supplied 18 hours after initial attachment to convert to cariogenic condition.

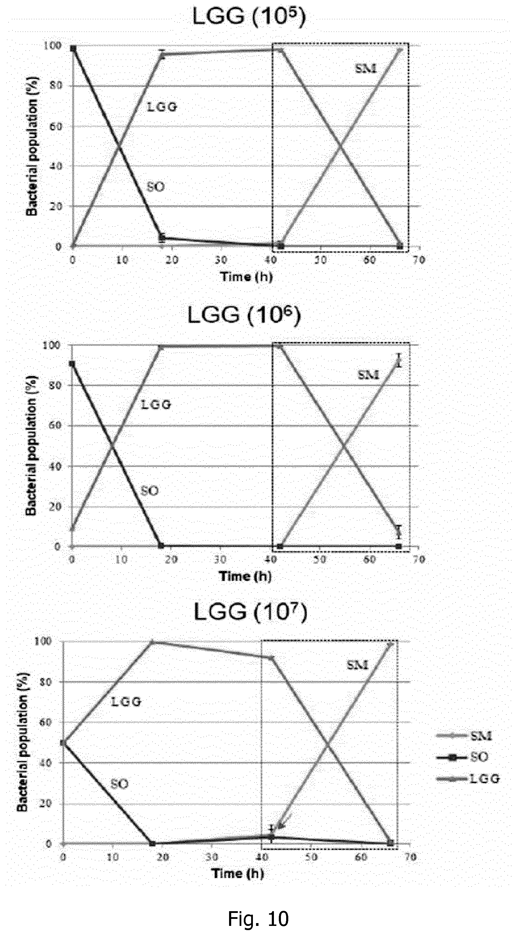

As shown in FIG. 10, the results indicate that the change in the LGG inoculum amount in the multi-species biofilm model did not significantly change the population shift between S. *mutans* and LGG. However, it was confirmed when the inoculum amount of LGG (10⁷) was large, *S. oralis* was detected in the biofilm that entered the maturation stage (*See* the red arrow in FIG. 10).

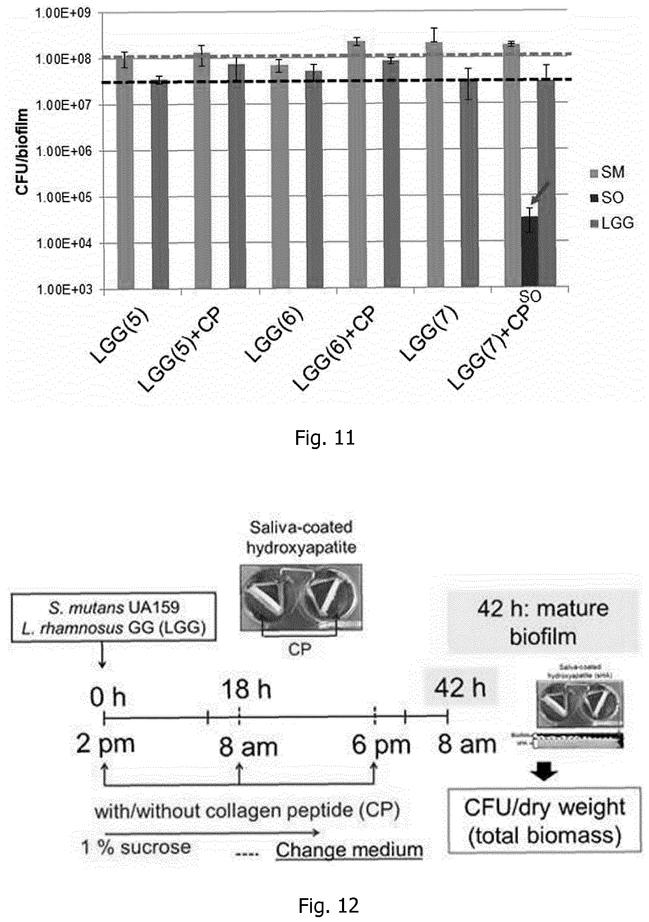

Next, the effect of the addition of collagen hydrolyzate (CP) on the growth of the strain in the biofilm in the change of the LGG inoculum amount was confirmed, and the results are shown in FIG. 11.

As a result, it was confirmed that the high inoculum amount of LGG and the addition of collagen hydrolysate had an effect on the growth of *S. oralis* while there was no significant change in the growth of *S. mutans* and LGG (*See* the red arrow in FIG. 11).

### Example 9. Confirmation of effect of combined treatment of LGG and collagen hydrolysate on biofilm production

The effect of treatment with collagen hydrolysate (5 mg/ml) on the formation of *S. mutans* biofilm and the effect of combined treatment with LGG (10⁷ CFU/ml) and collagen hydrolysate on the formation of *S. mutans* biofilm was verified with single biofilm and dual biofilm derived from the sHA disk model as shown in FIG. 12. After culturing the biofilm for 42 hours, the number of viable cells of *S. mutans* and LGG and the dry weight of the biofilm were confirmed in the same manner as in the above example. Collagen hydrolysate was added at the time of initial inoculation and at the time of changing the culture medium for 18 hours and 28 hours. LGG was inoculated and cultured at the same time as *S. mutans* was inoculated.

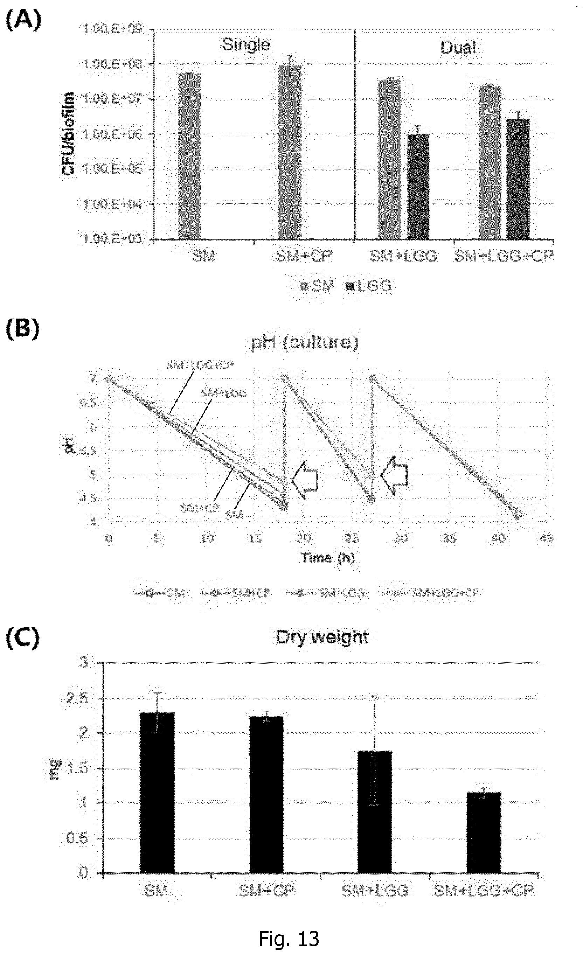

As shown in FIG. 13, the results indicate that the addition of collagen hydrolysate (CP) in the *S. mutans* single experimental group did not have a significant effect on biofilm production compared to the control group. However, it was confirmed that among them, LGG (10⁷)-added experimental group significantly inhibited biofilm by the addition of the collagen hydrolysate.

Further, after culture, the pH of the medium was measured. The results indicate that the pH of the medium was maintained to be relatively high compared to other groups.

Therefore, the above results indicate that the simultaneous treatment of LGG and collagen hydrolysate could significantly inhibit the formation of caries-inducing biofilms.

Collectively, the present invention confirms the synergistic effect of simultaneous treatment of *Lactobacillus rhamnosus* and collagen hydrolysate in inhibiting biofilm production generated by *Streptococcus mutans.* According to the present invention, although *Lactobacillus rhamnosus* can inhibit biofilm formation even as a single agent, the inhibitory ability is significantly increased when it is simultaneously treated with the collagen hydrolysate. The collagen hydrolysate inhibits acid production *of Lactobacillus rhamnosus* or *Streptococcus mutans.* Thus, the *Lactobacillus rhamnosus* and collagen hydrolysate can be usefully used as a composition for inhibiting the production of biofilms, an oral composition for inhibiting the biofilm or a composition for preventing, alleviating, or treating oral diseases caused by the biofilms.

## Claims

1. A composition for inhibiting biofilm, the composition comprising at least one selected from the group consisting of *Lactobacillus rhamnosus,* its culture medium, its extract and its cell-free supernatant; and collagen hydrolysate.

2. The composition of claim 1, wherein the *Lactobacillus rhamnosus* is *Lactobacillus rhamnosus* GG DSM 33156.

3. The composition of claim 2, wherein the *Lactobacillus rhamnosus* is included in a concentration of 1.0 × 10⁵ to 1.0 × 10¹² CFU (colony-forming unit)/ml.

4. The composition of claim 1, wherein the collagen hydrolysate has a molecular weight of 5000 Da or less.

5. The composition of claim 1, wherein the collagen hydrolyzate inhibits acid production of *Lactobacillus rhamnosus* or *Streptococcus mutans* strains.

6. The composition of claim 1, wherein the biofilm is produced by *Streptococcus mutans.*

7. The composition of claim 1, wherein the composition for inhibiting biofilm promotes growth of *Streptococcus oralis* in the biofilm.

8. An oral composition for inhibiting biofilm production, the composition comprising at least one selected from the group consisting of *Lactobacillus rhamnosus,* its culture medium, its extract and its cell-free supernatant; and collagen hydrolysate.

9. A pharmaceutical composition for preventing or treating oral diseases caused by *Streptococcus mutans,* the composition comprising at least one selected from the group consisting of *Lactobacillus rhamnosus,* its culture medium, its extract, and its cell-free supernatant; and collagen hydrolysate.

10. The pharmaceutical composition of claim 9, wherein the oral disease is caused by biofilm produced by *Streptococcus mutans.*

11. The pharmaceutical composition of claim 9, wherein the oral disease includes at least one selected from the group consisting of dental caries, periodontitis, stomatitis, gum recession, gum hypertrophy and gingivitis.

12. A food composition for preventing or alleviating oral diseases caused by *Streptococcus mutans,* the composition comprising at least one selected from the group consisting of *Lactobacillus rhamnosus,* its culture medium, its extract, and its cell-free supernatant; and collagen hydrolysate.

13. A feed additive composition for inhibiting biofilm production, the composition comprising at least one selected from the group consisting of *Lactobacillus rhamnosus,* its culture medium, its extract, and its cell-free supernatant; and collagen hydrolysate.

14. A method for inhibiting biofilm comprising the step of administering to an individual at least one selected from the group consisting *of Lactobacillus rhamnosus,* its culture medium, its extract and its cell-free supernatant; and collagen hydrolysate.

15. A method for treating globular disease caused by *Streptococcus mutans* comprises the step of administering to an individual at least one selected from the group consisting of *Lactobacillus rhamnosus,* its culture medium, its extract and its cell-free supernatant; and collagen hydrolysate.
